# EUROPEAN PATENT SPECIFICATION

(11) **EP 1 651 191 B1**
(45) Date of publication and mention of the grant of the patent: **12.11.2008**
(21) Application number: 04766204.4
(22) Date of filing: 13.07.2004
(51) Int. Cl.: A61K 9/20

(54) **EFFERVESCENT PHARMACEUTICAL COMPOSITIONS CONTAINING VITAMIN D, CALCIUM AND PHOSPATE AND THEIR THERAPEUTIC USE**
SPRUDELNDE PHARMAZEUTISCHE ZUSAMMENSETZUNGEN MIT VITAMIN D, CALCIUM UND PHOSPAT UND IHRE THERAPEUTISCHE VERWENDUNG
COMPOSITIONS PHARMACEUTIQUES EFFERVESCENTES CONTENANT DE LA VITAMINE D, DU CALCIUM ET DU PHOSPHATE ET LEUR UTILISATION THERAPEUTIQUE

(30) Priority: 17.07.2003 IT FI20030194
(43) Date of publication of application: 03.05.2006
(73) Proprietor: Menarini International Operations Luxembourg S.A., 1611 Luxembourg (LU)
(72) Inventor: TOSETTI, Alessandro, I-50012 Bagno a Ripoli (IT); VITI, Giovanni, I-50127 Firenze (IT)
(74) Representative: Gervasi, Gemma
(86) International application number: PCT/EP2004/051473
(87) International publication number: WO 2005/011639

(56) References cited:
- WO-A-99/06051
- WO-A-03/055500
- DATABASE CA [Online] CHEMICAL ABSTRACTS SERVICE, COLUMBUS, OHIO, US; LU, CHENGQIAN: "Effervescent tablet of balanced type nutrients and its preparation" XP002313083 retrieved from STN Database accession no. 2004:747868 & CN 1 408 363 A (SHIJIJINDA HEALTH ARTICLE CO L) 9 April 2003 (2003-04-09)

## Description

### FIELD OF THE INVENTION

This invention refers to an effervescent pharmaceutical composition comprising vitamin D combined with calcium and phosphate, useful in the treatment of nutritional deficiencies of calcium and vitamin D in the elderly.

### STATE OF THE ART

The use of vitamin D and calcium salts, separately or combined, for the treatment of various pathologies including those concerning the metabolism of calcium in the elderly, is already documented in the state of the art. It is equally well-known that phosphate can play an extremely important role in these pathologies (Lotz M et al New England J. of Medicine, 1968, 278, 409-415; Le Boff M S, J. American Medical Association, 1999, 28, 1505-1530).

In these formulations, to avoid the retention of liquids in patients who may be subject to this problem, it can also be important to fully or partially replace the sodium salts with the analogue potassium salts.

There are numerous examples of solid oral pharmaceutical formulations containing vitamin D, calcium and phosphate, both available on the market or simply described in the prior art.

For example, the French patent No. 2724844 (EP785769) describes compositions containing vitamin D and calcium salts; among the calcium salts referred to there is also phosphate, but the process used and the entire description are not suitable for obtaining an effervescent composition. In fact no suggestion exists for the use of an acid excipient, such as citric acid, which would produce effervescence. In addition the presence of the phosphate in said compositions is envisaged as a possible anion of the salt containing the calcium, but in this case there is no possibility of including the carbonate ion in the composition, which makes it furthermore impossible to obtain an effervescent formulation. Furthermore, the quantity of calcium ion in the compositions exemplified is relatively low, and no greater than approximately 500 mg per tablet.

The European patent No. 588 539 describes, on the other hand, compositions comprising derivatives of vitamin D2 or D3, and therefore compounds chemically different from vitamin D.

The French patent No. 2073271 deals with dermatological creams containing calcium gluconate and vitamin D2, but it is limited to forms for topical use and phosphate is never among the active ingredients taken into consideration.

The international patent application No. WO 98/41217 deals with formulations containing a calcium salt and vitamin D, but these formulations are in directly drinkable liquid form and no reference is ever made to phosphate.

The European patent No. 872 240 describes chewable tablets containing calcium phosphate and vitamin D, but these are never effervescent (see similar considerations made in the case of FR 2724844).

The international patent application No. WO 99/06051 claims compositions based on vitamin D and calcium salts, including phosphate, and characterised by the presence of some particular binding agents, such as propylene glycol and polyethylene glycols with low molecular weight, liquid paraffin or silicone oil, but it does not permit the production of effervescent tablets.

Furthermore, various effervescent pharmaceutical compositions exist on the market containing calcium ion and vitamin D, for example, in Italy, the pharmaceutical compositions sold under the brands Cacit^{®} Vitamina D3, Calcidon^{®} and Eurocal^{®}, but none of them also contain phosphate as the active ingredient.

Although there are numerous examples of pharmaceutical formulations based on calcium salts and vitamin D, there is still the need to find new solutions that meet therapeutic requirements without presenting problems.

When high doses of calcium have to be administered (e.g. from 1 to 2 g per dose) together with vitamin D and high doses of phosphate (e.g. from 1.5 to 3 g per dose) for prolonged periods, such as those required for the treatment of bone pathologies in the elderly, the problem of compliance must be taken into consideration.

Using an insoluble salt, such as calcium phosphate, it is highly likely that the patient will experience a marked and unpleasant "sand effect" which considerably reduces compliance.

In WO99/06051 it was claimed that this problem had been solved by the use of particular binding agents. A new method which solved this problem even more effectively was, surprisingly, recourse to effervescent formulations according to the present invention.

WO 03/055500 discloses compositions containing Vitamin D and calcium salts (i. a. calcium carbonate and calcium phosphate) discussing the possibility of providing effervescent compositions; the described compositions containing calcium phosphate are not effervescent.

Frequently, to avoid the "sand effect" caused by insoluble calcium salts, soluble salts of the same have been used such as gluconate, edetate, lactate, citrate etc..

In this way, however, to administer for example 1.2 g of calcium ion we would be obliged, given the relative molecular weights, to use approximately 17 g of calcium gluconate or approximately 13 g of calcium lactate pentahydrate; to this the weight of the salt containing the phosphate has to be added (e.g. sodium or potassium phosphate), contributing a further 3-4 g. In practice we would be obliged to use a considerable mass in weight of compounds, also administering ions not required in treatment of the pathology which, furthermore, given their large quantity, could also create problems for the patient, especially in the treatment of chronic cases.

This effect of an unnecessary increase in the mass of compounds occurs whatever calcium salt is used as an alternative to calcium phosphate.

Clearly costs can also undergo unjustified increase as a result of the increase in the quantity of products used.

None of the pharmaceutical compositions developed so far have proved to be capable of completely eliminating the problems described above.

### SUMMARY OF THE INVENTION

The Applicant has now surprisingly found a new effervescent pharmaceutical composition based on vitamin D and calcium phosphate, which completely eliminates the problems of compliance described above and which, using as calcium salt the calcium phosphate which contains both calcium and phosphate ions, also avoids the problem of the administration of unnecessary ions.

The subject of the present invention is therefore an oral pharmaceutical composition comprising vitamin D combined with a calcium salt, characterised by the fact that the composition is of the effervescent type, the calcium salt is tribasic calcium phosphate, and is present in micronised powder form so that 90% of the particles have a diameter of less than 30 micrometres in a concentration of between 1 and 2 g of calcium ion for each posological unit.

A further subject of the invention is use of the above-mentioned pharmaceutical composition in treatment of the pathological forms that involve the loss of bone mass in the elderly, such as osteoporosis, and the prevention of diseases connected with the metabolism of calcium in the elderly, such as fractures of the proximal femur and other non-vertebral fractures.

### DETAILED DESCRIPTION OF THE INVENTION

In the present invention the following are preferably used:
- Tribasic calcium phosphate in micronised powder form. Micronisation is such that the diameter of the particles is, for at least 90% of them, less than 30 micrometres, and preferably less than 15 micrometres.
- Vitamins D2 or D3 having concentration equal to 100 U.I. per mg of Vitamin D used.

For production of the effervescent composition of the invention, sodium bicarbonate, potassium bicarbonate or their mixtures are preferably added to the composition as the source of CO₂. The effervescent formulation obtained therefore contains: Ca₃ (PO₄)₂, NaHCO₃ (or KHCO₃ or their mixtures) and it is not necessary to add a quantity of carbonate or bicarbonate ions as the source of CO₂ in an equimolar quantity to the Ca ion. The same applies to the cation connected, whether sodium or potassium, and to the acid component that induces effervescence, which is preferably citric acid. Consequently, with the same concentrations of Ca and phosphate ions, this formulation necessarily has a lower concentration of other ions (such as sodium which, as is known in the prior art, it is always advisable to reduce) and a lower mass in weight with respect to the other possible effervescent formulations containing the same concentrations of calcium and phosphate (for example even with respect to a formulation of the type 2CaCO₃ + 3 Na₃PO₄ (or K₃PO₄ or similar)).

When dispersed in water, for administration to the patient, with the effervescent compositions produced according to the present invention, the calcium phosphate remains undissolved as a fine suspension which only slowly tends to deposit on the bottom. In general, once the effervescence has subsided, the precipitate on the bottom is negligible for the first 10-15 minutes, and is no higher than 10-15% of the total mass of the calcium phosphate.

Surprisingly, these formulations do not produce the "sand effect", are not unpleasant and are well-accepted by patients. With the present compositions, the mass of calcium phosphate is kept in suspension for up to 5 times longer than the times observed for similar non-effervescent formulations.

The behaviour observed for the present compositions and described above is surprising, as the calcium phosphate is insoluble and, due to its insolubility, we would have expected a low patient compliance, especially in the case of prolonged treatment, due to the "sand effect" associated with the insoluble residue of calcium phosphate.

According to a preferred form of embodiment of the invention, the present compositions, in addition to the active ingredients vitamin D and calcium phosphate, comprise the following pharmaceutically acceptable excipients considered for each individual posological unit:
- calcium ions such as calcium phosphate in a concentration between 1 and 2 g of calcium and, consequently, phosphate ions in a quantity between 1.58 and 3.2 g;
- vitamin D, preferably as vitamin D2 (ergocalciferol) or D3 (cholecalciferol) or mixtures of the two, in quantities between 500 and 1000 U.I. of vitamin D;
- sodium or potassium bicarbonate or a mixture of the two in a concentration of between 7 and 18 total mM;
- anhydrous citric acid in a quantity between 500 and 2000 mg;
- a binder in liquid form chosen from propylene glycol or polyethylene glycol with molecular weight between 300 and 1500, in a quantity between 300 and 2000 mg;
- one or more sweeteners with binder characteristics chosen from xylitol, mannitol or sorbitol in overall quantities of between 300 and 2000 mg.

More preferably, the effervescent pharmaceutical compositions produced according to the present invention have the following general composition, considered for each individual posological unit:
- calcium ions such as calcium phosphate in quantities between 1 and 2 g of calcium and, consequently, phosphate ions in quantities between 1.58 and 3.2 g;
- vitamin D, preferably as vitamin D2 (ergocalciferol) or D3 (cholecalciferol) or mixtures of the two, in concentrations of between 500 and 1000 U.I. of vitamin D;
- sodium or potassium bicarbonate or a mixture of the two, in a concentration of between 10.5 and 13 total mM;
- anhydrous citric acid in a quantity between 850 and 1650 mg;
- a binder in liquid form chosen from propylene glycol or polyethylene glycol with low molecular weight, for example with molecular weight of between 300 and 1500 (like Macrogol 400) in quantities of between 400 and 900 mg;
- one or more sweeteners with binder characteristics chosen from xylitol, mannitol or sorbitol in overall quantities of between 450 e 1200 mg;
- one or more non-sugar sweeteners chosen from saccharin, cyclamate, aspartame or their salts;
- any other excipients pharmaceutically acceptable according to those known in the state of the art, for example one or more of the following excipients:

- an aroma preferably chosen from orange or lemon aroma;
- an antioxidant preferably chosen from alpha, beta or gamma tocopherol;
- a binder, preferably chosen from polyvinyl pyrrolidone, hydroxypropylcellulose or hydroxyethylcellulose;
- an antifoaming agent such as simeticone;
- a surface-active agent chosen preferably from a polysorbate (such as polysorbate 20), sodium lauryl sulphate or dioctyl sulphosuccinate.

The above composition is suitable for the preparation of effervescent tablets or bags containing effervescent granulate.

Formulations comprising Mg salts also form part of the present invention, preferably magnesium bicarbonate or carbonate (in a percentage such that, if the carbonate is considered as corresponding to 2 equivalents of bicarbonate, the total bicarbonate is within the above-mentioned limits and the part due to the Mg salt is no higher than 30 of the total contribution).

The use of an effervescent granulate according to the present invention, once poured into water, permits rapid disintegration of the granulate with the formation of a fine dispersion which, surprisingly, does not produce the "sand effect" and is therefore not unpleasant for the patient and is readily usable.

To produce the formulations described, the methods commonly used for the preparation of effervescent formulations can be adopted. For example, the following dry granulation method can be used:
the tribasic calcium phosphate is mixed with the liquid binder in a granulator and calibrated. The vitamin D and all the other excipients are then added, mixing carefully.

According to the present invention with posological unit it is preferably intended a unit chosen in the group consisting of: a bag containing granulate, a double-section bag containing granulate, a tablet, a divisible tablet.

Examples of the present invention are the following.

### EXAMPLE 1

### Preparation of effervescent granulate according to the invention

### Composition for 7000 mg of granulate:

| | |
|---|---|
| Micronised powder tribasic calcium phosphate | 3100 mg |
| Sodium saccharin | 13 mg |
| Anhydrous citric acid | 1615 mg |
| Sodium bicarbonate | 1100 mg |
| Macrogol 400 | 490 mg |
| Cholecalciferol ( Vitamin D3 ) 100 U.I./mg | 8.8 mg |
| Xylitol | 485 mg |
| Sodium cyclamate | 100 mg |
| Polyvinyl pyrrolidone K30 | 88.2 mg |

### Preparation:

In a granulator the macrogol 400 is absorbed on the tribasic calcium phosphate and calibrated. The vitamin D3, sodium cyclamate, PVD K30, sodium saccharin, anhydrous citric acid, sodium bicarbonate and xylitol are added and mixed thoroughly.

### EXAMPLE 2

### Preparation of effervescent granulate according to the invention

### Composition for 7000 mg of granulate:

| | |
|---|---|
| Micronised powder tribasic calcium phosphate | 3100 mg |
| Sodium saccharin | 15 mg |
| Anhydrous citric acid | 1100 mg |
| Sodium bicarbonate | 1100 mg |
| Propylene glycol | 490 mg |
| Cholecalciferol (Vitamin D3) 100 U.I./mg | 8.8 mg |
| Alpha-tocopherol | 7 mg |
| Orange aroma | 120 mg |
| Mannitol | q.s. to 7000 mg |

### Preparation:

The effervescent granulate was prepared with the above composition via the same procedure as the one described above in Example 1.

### EXAMPLE 3

### Preparation of effervescent granulate according to the invention

### Composition for 7000 mg of granulate:

| | |
|---|---|
| Micronised powder tribasic calcium phosphate | 3100 mg |
| Sodium saccharin | 13 mg |
| Anhydrous citric acid | 850 mg |
| Sodium bicarbonate | 900 mg |
| Macrogol 400 | 800 mg |
| Cholecalciferol ( Vitamin D3 ) 100 U.I./mg | 8.8 mg |
| Lemon aroma | 100 mg |
| Mannitol | q.s. to 7000 mg |

### Preparation:

The effervescent granulate was prepared with the above composition via the same procedure as the one described above in Example 1.

The pharmaceutical compositions according to the present invention are suitable for the continuous treatment, even for very long periods, according to the directions of the doctor in charge, of nutritional deficiency of calcium and vitamin D in the elderly, to reduce age-related bone loss and prevent fractures of the femur and other non-vertebral fractures.

The recommended dosage, according to the directions of the doctor in charge, should be one-two posological units per day.

These pharmaceutical compositions are applied also for the prevention of osteoporosis induced by corticosteroid treatment according to procedures similar to those described for the preceding pathologies.

## Claims

1. Oral pharmaceutical composition comprising vitamin D combined with a calcium phosphate, **characterised in that** the composition is of the effervescent type and the calcium phosphate is present as tribasic calcium phosphate in micronised powder form so that 90% of the particles have a diameter of less than 30 micrometres in a concentration of between 1 and 2 g of calcium ion per posological unit.

2. Pharmaceutical composition according to claim 1, in which said tribasic calcium phosphate is micronised so that 90% of the particles have a diameter of less than 15 micrometres.

3. Pharmaceutical composition according to claims 1 and 2, in which the vitamin D used is chosen from vitamin D2 (or ergocalciferol), vitamin D3 (or cholecalciferol) or their mixtures.

4. Pharmaceutical composition according to claim 3, in which the vitamin D used is vitamin D3 (or cholecalciferol).

5. Pharmaceutical composition according to claims 1 and 2, in which the concentration of vitamin D per posological unit is between 500 and 1000 U.I.

6. Pharmaceutical composition according to claims 1-5, furthermore comprising sodium or potassium bicarbonate or a mixture of the two, in a concentration of between 7 and 18 mM per posological unit.

7. Pharmaceutical composition according to claims 1-6, furthermore comprising citric acid in a concentration of between 500 and 2000 mg per posological unit.

8. Pharmaceutical composition according to claims 1-7, furthermore comprising a binding agent in liquid form chosen from propylene glycol and polyethylene glycols with molecular weight of between 300 and 1500, in concentrations of between 300 and 2000 mg per posological unit.

9. Pharmaceutical composition according to claims 1-8, furthermore comprising one or more sweetening binders chosen from xylitol, mannitol or sorbitol, in an overall concentration of between 300 and 2000 mg per posological unit.

10. Pharmaceutical composition according to claims 1-9, with the following composition:
- tribasic calcium phosphate in a concentration of between 1000 and 2000 mg of calcium ion and, consequently, between 1600 and 3200 mg of phosphate ion;
- vitamin D3 (or cholecalciferol) in concentrations of 500-1000 U.I. of vitamin D;
- sodium bicarbonate or potassium bicarbonate or a mixture of the two in a quantity equal to 10-14 mM;
- anhydrous citric acid in a quantity between 850 and 1650 mg;
- a binder in liquid-form chosen from propylene glycol or polyethylene glycol with molecular weight of between 300 and 1500, in a quantity between 400 and 900 mg;
- one or more sweeteners with binder characteristics chosen from xylitol, mannitol or sorbitol in overall concentrations of 450 to 1200 mg;
- one or more non-sugar sweeteners chosen from saccharin, cyclamate or aspartame or their salts;
- any other pharmaceutically acceptable excipients.

11. Pharmaceutical composition according to claims 1-10, **characterised by** the possible presence of Mg ions introduced as bicarbonate or carbonate salt in a concentration such that, considering the carbonate as corresponding to 2 bicarbonate equivalents, the total bicarbonate is within the above limits of claim 5 and the part due to the Mg salt is no higher than 30 of the total contribution.

12. Pharmaceutical composition according to claims 1-11, in the form of effervescent tablets or effervescent granulate.

13. Pharmaceutical composition according to claim 1 in which, after dissolution in water, said calcium phosphate remains as a fine suspension for a period of at least 10-15 minutes after subsidence of the effervescence.

14. Use of the pharmaceutical composition as described in claims 1-13 in the manufacture of a medicament, for the prevention of osteoporosis induced by corticosteroid treatment.

15. Use of the pharmaceutical composition as described in the claims from 1-13 in the manufacture of a medicament, for the treatment of nutritional deficiency of calcium and vitamin D in the elderly, to reduce age-related bone loss and prevent fractures of the femur and other non-vertebral fractures.

## Patentansprüche

1. Orale pharmazeutische Zusammensetzung, enthaltend Vitamin D in Kombination mit einem Calciumphosphat, **dadurch gekennzeichnet, dass** die Zusammensetzung vom Brausetyp ist und das Calciumphosphat in einer Konzentration zwischen 1 und 2 g Calciumionen pro posologischer Einheit als tribasisches Calciumphosphat in mikronisierter Pulverform vorliegt, derart, dass 90 % der Partikel einen Durchmesser von weniger als 30 µm aufweisen.

2. Pharmazeutische Zusammensetzung gemäss Anspruch 1, in der das tribasische Calciumphosphat mikronisiert ist, derart, dass 90 % der Partikel einen Durchmesser von weniger als 15 µm besitzen.

3. Pharmazeutische Zusammensetzung gemäss den Ansprüchen 1 und 2, in der das verwendete Vitamin D ausgewählt ist aus Vitamin D2 (oder Ergocalciferol), Vitamin D3 (oder Cholecalciferol) oder Mischungen davon.

4. Pharmazeutische Zusammensetzung gemäss Anspruch 3, in der das verwendete Vitamin D Vitamin D3 (oder Cholecalciferol) ist.

5. Pharmazeutische Zusammensetzung gemäss den Ansprüchen 1 und 2, in der die Konzentration an Vitamin D pro posologischer Einheit zwischen 500 und 1.000 U.I. beträgt.

6. Pharmazeutische Zusammensetzung gemäss den Ansprüchen 1 bis 5, ferner enthaltend Natrium- oder Kaliumbicarbonat oder eine Mischung der beiden in einer Konzentration zwischen 7 und 18 mM pro posologischer Einheit.

7. Pharmazeutische Zusammensetzung gemäss den Ansprüchen 1 bis 6, ferner enthaltend Zitronensäure in einer Konzentration zwischen 500 und 2.000 mg pro posologischer Einheit.

8. Pharmazeutische Zusammensetzung gemäss den Ansprüchen 1 bis 7, ferner enthaltend ein Bindemittel in flüssiger Form, ausgewählt aus Propylenglykol und Polyethylenglykolen, mit einem Molekulargewicht zwischen 300 und 1.500 in Konzentrationen zwischen 300 und 2.000 mg pro posologischer Einheit.

9. Pharmazeutische Zusammensetzung gemäss den Ansprüchen 1 bis 8, ferner enthaltend ein oder mehrere süssende Bindemittel, ausgewählt aus Xylitol, Mannitol oder Sorbitol, in einer Gesamtkonzentration zwischen 300 und 2.000 mg pro posologischer Einheit.

10. Pharmazeutische Zusammensetzung gemäss den Ansprüchen 1 bis 9 mit der folgenden Zusammensetzung:
- tribasisches Calciumphosphat in einer Konzentration zwischen 1.000 und 2.000 mg an Calciumionen und folglich zwischen 1.600 und 3.200 mg an Phosphationen;
- Vitamin D3 (oder Cholecalciferol) in Konzentrationen von 500 bis 1.000 U.I. an Vitamin D;
- Natriumbicarbonat oder Kaliumbicarbonat oder eine Mischung der beiden in einer Menge, die 10 bis 14 mM entspricht;
- wasserfreie Zitronensäure in einer Menge zwischen 850 und 1.650 mg;
- ein Bindemittel in flüssiger Form, ausgewählt aus Propylenglykol oder Polyethylenglykol mit einem Molekulargewicht zwischen 300 und 1.500 in einer Menge zwischen 400 und 900 mg;
- ein oder mehrere Süssungsmittel mit Bindemitteleigenschaften, ausgewählt aus Xylitol, Mannitol oder Sorbitol, in einer Gesamtkonzentrationen von 450 bis 1.200 mg;
- ein oder mehrere Süssungsmittel, die keine Zucker sind, ausgewählt aus Saccharin, Cyclamat oder Aspartam oder deren Salzen;
- beliebige andere pharmazeutisch akzeptable Hilfsstoffe.

11. Pharmazeutische Zusammensetzung gemäss den Ansprüchen 1 bis 10, **gekennzeichnet durch** die mögliche Gegenwart von Magnesiumionen, die als Bicarbonat- oder Carbonatsalze in einer solchen Konzentration eingeführt werden, dass, wenn das Carbonat als zwei Bicarbonat-Äquivalenten entsprechend gerechnet wird, das gesamte Bicarbonat innerhalb der oben in Anspruch 5 genannten Grenzen liegt, und der Teil, der vom Magnesiumsalz herrührt, nicht mehr als 30% der Gesamteinbringung beträgt.

12. Pharmazeutische Zusammensetzung gemäss den Ansprüchen 1 bis 11 in Form von Brausetabletten oder Brausegranulat.

13. Pharmazeutische Zusammensetzung gemäss Anspruch 1, in der nach Auflösung in Wasser das Calciumphosphat als feine Suspension für einen Zeitraum von mindestens 10 bis 15 Minuten nach Ende des Sprudelns bestehen bleibt.

14. Verwendung der pharmazeutischen Zusammensetzung, wie in den Ansprüchen 1 bis 13 beschrieben, bei der Herstellung eines Medikaments zur Vorbeugung von durch Corticosteroidbehandlung induzierter Osteoporose.

15. Verwendung einer pharmazeutischen Zusammensetzung, wie in den Ansprüchen 1 bis 13 beschrieben, bei der Herstellung eines Medikaments zur Behandlung von Nährstoffmangel an Calcium und Vitamin D bei älteren Menschen, um den altersbedingten Knochenverlust zu verringern und Frakturen der Oberschenkelknochen und anderen Nicht-Wirbelfrakturen zu verhindern.

## Revendications

1. Composition pharmaceutique orale comprenant de la vitamine D combinée avec un phosphate de calcium, **caractérisée en ce que** la composition est de type effervescent et le phosphate de calcium est présent en tant que phosphate de calcium tribasique sous forme de poudre micronisée de sorte que 90 % des particules aient un diamètre inférieur à 30 micromètres, en une concentration de 1 à 2 g d'ion calcium par unité posologique.

2. Composition pharmaceutique selon la revendication 1, dans laquelle ledit phosphate de calcium tribasique est micronisé de sorte que 90 % des particules aient un diamètre inférieur à 15 micromètres.

3. Composition pharmaceutique selon la revendication 1 ou 2, dans laquelle la vitamine D utilisée est choisie parmi la vitamine D2 (ou ergocalciférol), la vitamine D3 (ou cholécalciférol) ou leurs mélanges.

4. Composition pharmaceutique selon la revendication 3, dans laquelle la vitamine D utilisée est la vitamine D3 (ou cholécalciférol).

5. Composition pharmaceutique selon la revendication 1 ou 2, dans laquelle la concentration en vitamine D par unité posologique est comprise entre 500 et 1000 U.I.

6. Composition pharmaceutique selon l'une quelconque des revendications 1 à 5, comprenant en outre du bicarbonate de sodium ou de potassium ou un mélange des deux, en une concentration comprise entre 7 et 18 mM par unité posologique.

7. Composition pharmaceutique selon l'une quelconque des revendications 1 à 6, comprenant en outre de l'acide citrique en une concentration comprise entre 500 et 2000 mg par unité posologique.

8. Composition pharmaceutique selon l'une quelconque des revendications 1 à 7, comprenant en outre un agent liant sous une forme liquide choisi parmi le propylène glycol et les polyéthylène glycols ayant un poids moléculaire compris entre 300 et 1500, en des concentrations comprises entre 300 et 2000 mg par unité posologique.

9. Composition pharmaceutique selon l'une quelconque des revendications 1 à 8, comprenant en outre un ou plusieurs liants édulcorants choisis parmi le xylitol, le mannitol ou le sorbitol, en une concentration globale comprise entre 300 et 2000 mg par unité posologique.

10. Composition pharmaceutique selon l'une quelconque des revendications 1 à 9, ayant la composition suivante :
- phosphate de calcium tribasique en une concentration comprise entre 1000 et 2000 mg d'ion calcium et, par conséquent entre 1600 et 3200 mg d'ion phosphate ;
- vitamine D3 (ou cholécalciférol) en des concentrations comprises entre 500 et 1000 U.I de vitamine D ;
- bicarbonate de sodium ou bicarbonate de potassium ou un mélange des deux en une quantité équivalente à 10 à 14 mM ;
- acide citrique anhydre en une quantité comprise entre 850 et 1650 mg ;
- liant sous forme liquide choisi parmi le propylène glycol ou le polyéthylène glycol ayant un poids moléculaire compris entre 300 et 1500, en une quantité comprise entre 400 et 900 mg ;
- un ou plusieurs édulcorants ayant des caractéristiques liantes choisis parmi le xylitol, le mannitol ou le sorbitol, en des concentrations globales comprises entre 450 et 1200 mg ;
- un ou plusieurs édulcorants autres que des sucres choisis parmi la saccharine, le cyclamate ou l'aspartame ou leurs sels ;
- n'importe quels autres excipients pharmaceutiquement acceptables.

11. Composition pharmaceutique selon l'une quelconque des revendications 1 à 10, **caractérisée par** la présence possible d'ions Mg introduits sous forme de sel de bicarbonate ou de carbonate en une concentration telle que, en considérant le carbonate comme correspondant à 2 équivalent de bicarbonate, le bicarbonate total est dans les limites ci-dessus de la revendication 5 et la partie due au sel de Mg n'est pas supérieure à 30 de la contribution totale.

12. Composition pharmaceutique selon l'une quelconque des revendications 1 à 11, sous la forme de comprimés effervescents ou de granulés effervescents.

13. Composition pharmaceutique selon la revendication 1, dans laquelle, après la dissolution dans de l'eau, ledit phosphate de calcium reste sous forme de suspension fine pendant une période d'au moins 10 à 15 minutes après réduction de l'effervescence.

14. Utilisation de la composition pharmaceutique selon l'une quelconque des revendications 1 à 13, dans la fabrication d'un médicament destiné à la prévention de l'ostéoporose induite par un traitement corticoïde.

15. Utilisation de la composition pharmaceutique selon l'une quelconque des revendications 1 à 13, dans la fabrication d'un médicament destiné au traitement d'une carence nutritionnelle en calcium et vitamine D chez les personnes âgées, pour réduire la perte osseuse liée à l'âge et empêcher les fractures du fémur et d'autres fractures non vertébrales.
